# FASCICULE DE BREVET EUROPEEN

(11) **EP 2 589 354 B1**
(45) Date de publication et mention de la délivrance du brevet: **23.04.2014**
(21) Numéro de dépôt: 12191012.9
(22) Date de dépôt: 02.11.2012
(51) Int. Cl.: A61F 2/46, A61B 17/17, A61B 17/15

(54) **Ensemble d'instrumentation chirurgicale pour poser une prothèse de cheville**
Gesamtheit der chirurgischen Instrumente zum Einsetzen einer Knöchelprothese
Surgical instrument assembly for inserting an ankle prosthesis

(30) Priorité: 04.11.2011 US 201161555593 P; 06.02.2012 FR 1251091
(43) Date de publication de la demande: 08.05.2013
(73) Titulaire: Tornier, 38330 Montbonnot-Saint-Martin (FR)
(72) Inventeur: Coulange, Vincent, 69003 Lyon (FR); Genna, Sophie, 38610 Venon (FR); Brunnarius, Yann, 26300 Chatuzange Le Goubet (FR); Lizee, Emmanuel, 38330 Saint Ismier (FR); Perineau, Christophe, 38000 Grenoble (FR); Bonnin, Michel, 69002 Lyon (FR); Coetzee, J. Christiaan, St. Paul, Minnesota 55122 (US); Colombier, Jean-Alain, 31130 Balma (FR); Judet, Thierry, 92410 Ville d'Avray (FR); Myerson, Mark, Baltimore, Maryland 21210 (US)
(74) Mandataire: Grand, Guillaume

(56) Documents cités:
- WO-A1-2009/158522
- GB-A- 2 445 146
- GB-A- 2 479 899
- US-A1- 2010 057 216

## Description

La présente invention concerne un ensemble d'instrumentation chirurgicale pour poser une prothèse de cheville.

La mise en place d'une prothèse articulaire au niveau de la cheville d'un patient nécessite, lors d'une intervention chirurgicale, typiquement par voie d'abord antérieure, de préparer, notamment par des résections, l'extrémité inférieure du tibia, ainsi que, souvent, l'extrémité supérieure de l'astragale du patient, pour y fixer à demeure les implants tibial et astragalien appartenant à la prothèse de cheville. En pratique, une fois que ces préparations osseuses sont effectuées, le chirurgien a fréquemment recours à des fantômes des implants prothétiques, lui permettant de s'assurer que ces préparations sont convenables et que des dégagements osseux supplémentaires ou des resurfaçages additionnels ne sont pas nécessaires. Ces fantômes ne sont pas nécessairement de la même forme que l'implant correspondant.

Dans le prolongement précis des considérations qui précèdent, WO-A-2009/158522 divulgue une instrumentation chirurgicale pour poser une prothèse de cheville, du type de celle définie dans le préambule de la revendication 1 annexée. En particulier, en lien avec les paragraphes [0173] à [0175] et avec les figures 34 et 35 de ce document, ce dernier divulgue, d'une part, un fantôme astragalien qui est conçu pour être mis en place sur la face supérieure, préalablement préparée, de l'astragale, typiquement à des fins de vérification de cette préparation préalable, ce fantôme astragalien étant pourvu d'ouvertures traversantes à travers lesquelles des outils de préparation osseuse peuvent être introduits, et, d'autre part, un fantôme tibial qui est conçu pour être fixé au tibia, et ce alors que le fantôme astragalien est toujours en place sur l'astragale : comme montré sur la figure 35 de WO-A-2009/158522, les fantômes astragalien et tibial coopèrent alors mécaniquement l'un avec l'autre de manière mobile, plus précisément de manière articulaire, cette coopération articulée étant ainsi mise en oeuvre pour vérifier le positionnement relatif des fantômes astragalien et tibial.

Ce qui vient d'être rappelé jusqu'ici s'applique aussi bien aux prothèses de cheville de première pose, qu'aux prothèses de cheville de révision, avec la contrainte supplémentaire pour ces dernières que l'implant astragalien est pourvu d'une quille d'ancrage astragalo-calcanéen, autrement dit une quille d'ancrage osseux qui est prévue suffisamment longue pour stabiliser l'implant astragalien vis-à-vis, à la fois, de l'astragale et du calcanéum du patient. Ceci étant dit, ce type de prothèse de cheville à quille longue peut bien entendu être posé en première intention, notamment si les os du pied sont très abimés. On comprend que, contrairement à l'instrumentation de WO-A-2009/158522 qui ne concerne que les prothèses de cheville à implant astragalien pourvu d'une quille d'ancrage courte, l'instrumentation de pose pour une prothèse de cheville à quille longue doit permettre au chirurgien de préparer en conséquence l'astragale et le calcanéum, pour que ces os soient prêts à recevoir la quille longue précitée dans un logement ad hoc. De plus, malgré tout le soin que peut apporter le chirurgien dans la manipulation d'une telle instrumentation, moyennant notamment une durée d'intervention significative, les risques ne sont pas négligeables que la préparation du logement précité ne soit pas satisfaisante, dans le sens où l'implantation du composant astragalien en résultant ne permet pas une bonne coopération articulaire ultérieure avec le composant tibial fixé au tibia.

Le but de la présente invention est de proposer un ensemble d'instrumentation chirurgicale pour poser une prothèse de cheville à implant astragalien pourvu d'une quille d'ancrage astragalo-calcanéen, ensemble d'instrumentation qui permet au chirurgien d'assurer un positionnement d'implantation satisfaisant entre l'implant tibial et l'implant astragalien, notamment pour des raisons de stabilité et de pérennité de la prothèse de cheville.

A cet effet, l'invention a pour objet un ensemble d'instrumentation chirurgicale pour poser une prothèse de cheville, ladite prothèse de cheville incluant un implant tibial et un implant astragalien pourvu d'une quille d'ancrage astragalo-calcanéen, cet ensemble d'instrumentation étant tel que défini à la revendication 1.

Une des idées à la base de l'invention est de lier mécaniquement le fantôme tibial et le guide de visée de manière que le positionnement vertical et médio-latéral du fantôme tibial sur le tibia contraint de manière correspondante le positionnement vertical et médio-latéral du guide visée. De cette façon, la mise en place, par ce guide de visée, d'un élément d'instrumentation astragalo-calcanéen, tel qu'une broche ou similaire, est positionnée par rapport au fantôme tibial alors que celui-ci est fixé au tibia dans la même configuration que sera ensuite fixé l'implant tibial de la prothèse de cheville. Une fois que cet élément d'instrumentation est ainsi mise en place à travers l'astragale et le calcanéum, il est prévu pour être utilisé par le chirurgien pour préparer l'astragale et la calcanéum à recevoir la quille d'ancrage de l'implant astragalien de la prothèse, cette préparation étant donc positionnée de manière fiable et précise par rapport au fantôme tibial fixé au tibia.

En pratique, l'élément d'instrumentation précité est mis en place par le guide de visée alors que le chirurgien fige la cheville du patient dans une configuration préférentielle, notamment en figeant le pied à 90° par rapport à la jambe du patient dans le plan sagittal. Ainsi, l'implantation de cet élément et donc, in fine, l'implantation de la quille d'ancrage de l'implant astragalien sont réalisées, grâce à l'instrumentation conforme à l'invention, en tenant compte de l'implantation de l'implant tibial et de la configuration préférentielle précitée de la cheville.

Les performances articulaires ultérieures de la prothèse de cheville ainsi implantée sont remarquables en raison du positionnement relatif satisfaisant des implants tibial et astragalien, ce positionnement étant obtenu facilement et rapidement par le chirurgien au cours de l'intervention de pose grâce à l'instrumentation conforme à l'invention.

Des caractéristiques additionnelles avantageuses de l'ensemble d'instrumentation conforme à l'invention, prises isolément ou selon toutes les combinaisons techniquement possibles, sont spécifiées aux revendications dépendantes 2 à 10.

L'invention sera mieux comprise à la lecture de la description qui va suivre, donnée uniquement à titre d'exemple et faite en se référant aux dessins sur lesquels :
- les figures 1 à 3 illustrent une prothèse de cheville, la figure 1 étant à une perspective éclatée tandis que les figures 2 et 3 correspondent à des élévations selon respectivement les flèches II et III de la figure 1 ;
- la figure 4 est une vue en perspective de la cheville d'un patient illustrant une première étape de préparation de cette cheville en vue de poser la prothèse des figures 1 à 3, cette première étape de préparation étant réalisée à l'aide d'une partie correspondante d'un exemple de réalisation d'un ensemble d'instrumentation conforme à l'invention ;
- la figure 5 est une vue analogue à la figure 4, illustrant une deuxième étape de préparation de la cheville à l'aide d'une partie correspondante de l'instrumentation ;
- la figure 6 est une vue analogue aux figures 4 et 5, illustrant une troisième étape de préparation de la cheville à l'aide d'une partie correspondante de l'instrumentation ;
- les figures 7 à 9 sont des vues similaires aux figures 4 à 6, illustrant respectivement des moments successifs d'une quatrième étape de préparation de la cheville à l'aide de parties correspondantes de l'instrumentation ;
- la figure 10 est une vue identique à la figure 9, avec coupe partielle selon le plan X indiqué à la figure 9 ;
- les figures 11 à 13 sont des vues similaires aux figures 4 à 9, illustrant respectivement des moments successifs d'une cinquième étape de préparation de la cheville à l'aide de parties correspondantes de l'instrumentation ;
- la figure 14 est une vue analogue aux figure 4 à 9 et 11 à 13, illustrant l'implantation de la prothèse des figures 1 à 3 au niveau de la cheville ayant été préparée par l'instrumentation ; et
- les figures 15, 16 et 17 sont des vues respectivement similaires aux figures 7, 9 et 10, illustrant une variante de l'instrumentation conforme à l'invention.

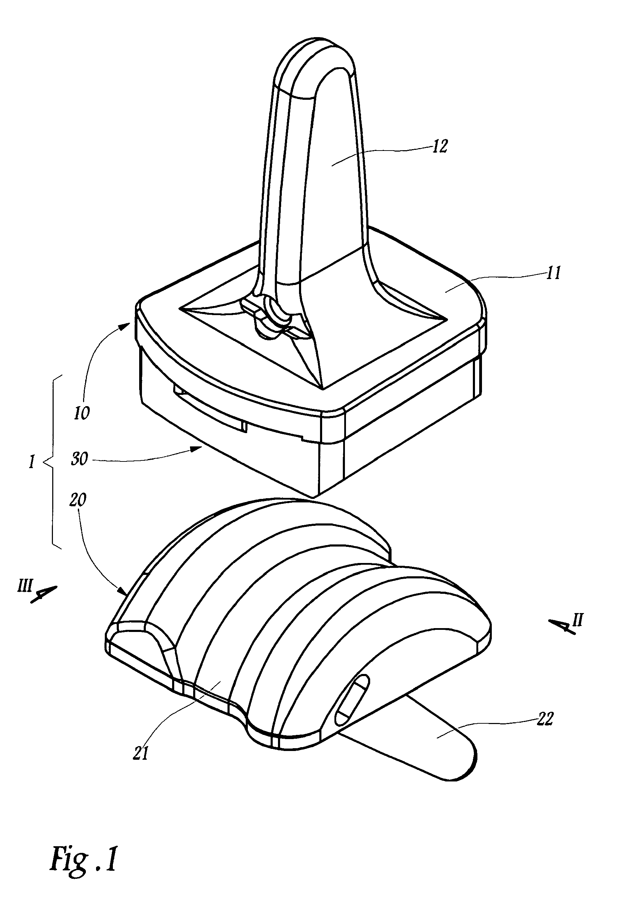

Sur les figures 1 à 3 est représenté un exemple d'une prothèse de cheville 1. Cette prothèse comporte trois composants distincts à implanter au niveau de l'articulation d'une cheville d'un être humain, en l'occurrence, pour l'exemple considéré sur les figures, une cheville droite d'un tel être humain. Ces composants sont un implant tibial 10, un implant astragalien 20 et un patin prothétique 30.

Par commodité, la suite de la description est orientée par rapport aux os d'une cheville dans leur position anatomique, c'est-à-dire que les termes « postérieur » ou « arrière », « antérieur » ou « avant », « droit », « gauche », « supérieur », « inférieur », etc. s'entendent par rapport à la cheville d'un patient se tenant debout sur ses pieds sur une surface sensiblement horizontale. De même, le terme « sagittal » correspond à une direction dans le sens antéro-postérieur, verticalement sur la ligne médiane de la cheville, tandis que le « plan frontal » correspond à un plan vertical perpendiculaire au plan sagittal de la cheville.

L'implant tibial 10 comporte une plaque 11 à fixer à l'extrémité inférieure du tibia droit d'un patient, après une préparation convenable de cette extrémité. A cet effet, la plaque 11 est, sur son côté supérieur, venue de matière avec une aile sagittale d'ancrage osseux 12 destinée à être cimentée par rapport au tibia. A titre de variante non représentée, d'autres moyens d'ancrage osseux, que l'aile sagittale 12, peuvent être envisagés pour la plaque 11 du moment qu'ils immobilisent efficacement l'implant tibial 10 à l'extrémité inférieure du tibia.

Sur son côté inférieur, la plaque 11 est solidarisée à la face supérieure du patin 30. En pratique, diverses formes de réalisation sont envisageables concernant l'interface, non visible en détail sur les figures, de solidarisation, fixe ou mobile, entre la plaque 11 et le patin 30, ces diverses formes de réalisation n'étant pas limitatives de la présente invention.

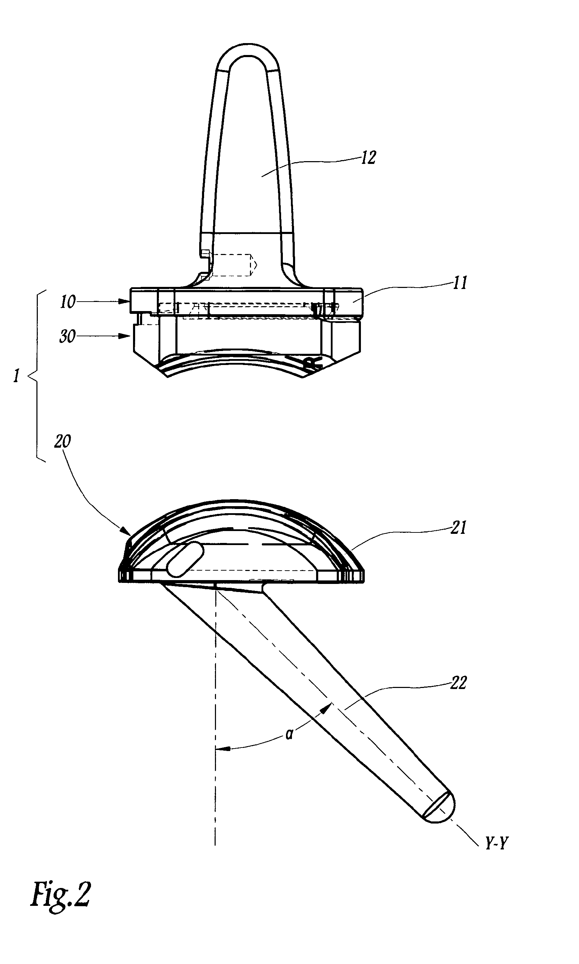

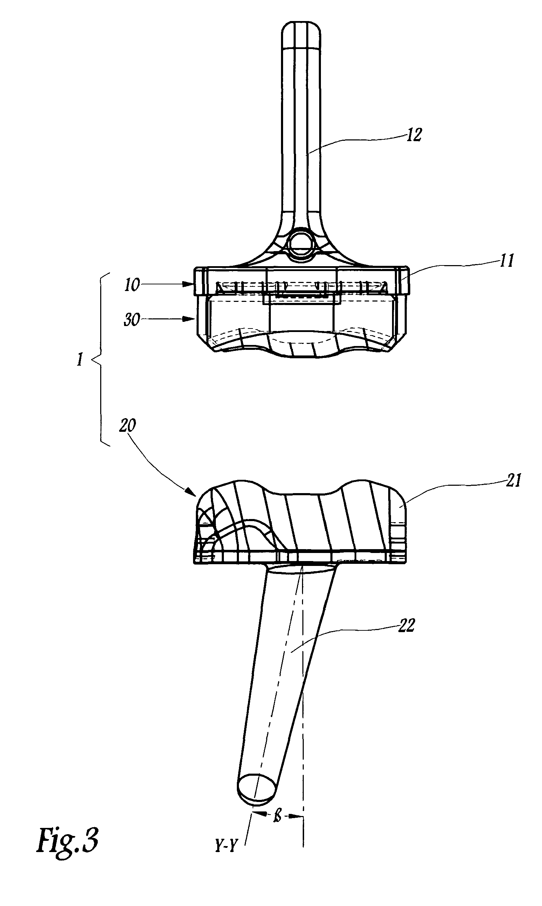

L'implant astragalien 20 comporte un bloc principal 21 à fixer à l'extrémité supérieure de l'astragale droit d'un patient, et ce par une quille d'ancrage 22 s'étendant vers le bas depuis le côté inférieur du bloc 21. Cette quille 22 présente une dimension longitudinale significative, dans le sens où, en service, cette quille 22 est prévue pour s'étendre continûment à travers tout l'astragale droit du patient et à travers au moins une partie du calcanéum droit du patient. De plus, comme bien visible sur les figures 2 et 3, cette quille 22 s'étend depuis le côté inférieur du bloc 21 avec une inclinaison spécifique, liée au fait que la quille 22 est avantageusement conçue pour s'étendre dans des parties a priori massives et résistantes de l'astragale et du calcanéum, notamment à des fins de stabilisation de cette quille vis-à-vis des os. L'intérêt de cette disposition est lié au fait que l'astragale du patient peut, au moment de la pose de l'implant 20, soit être de mauvaise qualité osseuse, soit n'être présente qu'en faible quantité, soit avoir préalablement été au moins en partie altérée, en particulier suite à la dépose d'un implant astragalien d'une prothèse de cheville de première pose, antérieurement implantée chez le patient à opérer, ce qui justifie d'ailleurs dans ce dernier cas que la prothèse 1 soit qualifiée de « prothèse de révision ».

A titre d'exemple préférentiel, mais non limitatif, la quille 22 présente une surface extérieure essentiellement tronconique, centrée sur un axe géométrique Y-Y qui s'étend suivant la direction longitudinale de la quille 22 et qui constitue un axe d'implantation de cette quille dans l'astragale et le calcanéum. Egalement à titre d'exemple non limitatif, et indépendamment de la forme extérieure tronconique de la quille 22, l'axe longitudinal central Y-Y de cette quille est incliné par rapport à la normale au côté inférieur du bloc principal 21, plus généralement par rapport à une direction anatomique verticale, en formant, dans un plan sagittal comme à la figure 2, une angulation α et en formant, dans un plan frontal comme à la figure 3, une angulation β. Les angulations α et β peuvent présenter des valeurs respectives variables selon l'anatomie du patient : en particulier, l'angulation α vaut de 40 à 55° environ et l'angulation β vaut de 0 à 15° environ.

Sur son côté supérieur, le bloc 21 délimite une surface articulaire destinée à coopérer avec une surface articulaire conjuguée qui est délimitée sur le côté inférieur du patin 30. En pratique, le profil des surfaces articulaires précitées n'est pas limitatif de la présente invention et ne sera donc pas décrit plus avant : dans tous les cas, par coopération articulaire entre le côté supérieur du bloc 21 et le côté inférieur du patin 30, la prothèse de cheville 1 procure avantageusement une cinématique proche de celle de l'articulation naturelle de la cheville.

On va décrire ci-après une méthode chirurgicale visant à implanter la prothèse de cheville 1, étant entendu que la prothèse considérée n'est qu'un exemple illustratif non limitatif de la méthode et des instruments chirurgicaux utilisés pour implanter cette prothèse. Autrement dit, la méthode et les instruments détaillés ci-après peuvent être utilisés pour implanter des prothèses de cheville de structure très diverses, par exemple dont les implants tibiaux et/ou astragaliens sont constitués de plusieurs parties assemblées les unes aux autres, de nature métallique, plastique et/ou céramique.

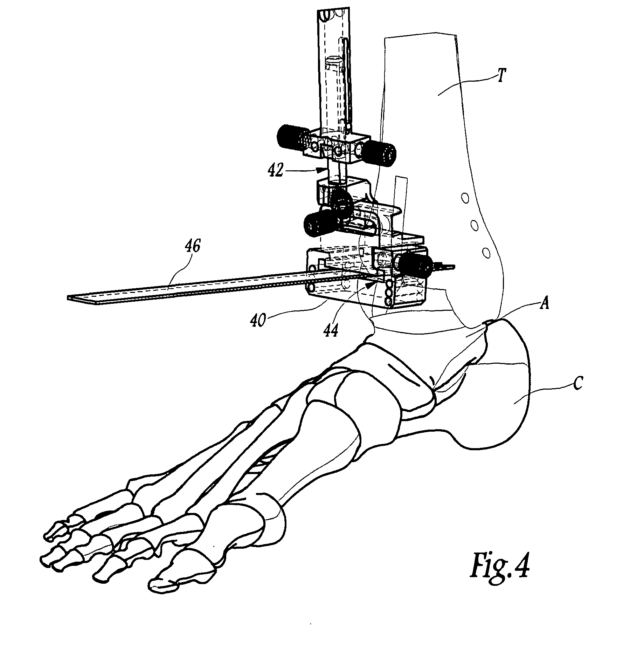

Dans une première étape opératoire, qui est représentée à la figure 4, le tibia T commence d'être préparé au niveau de la cheville droite du patient. A cet effet, le chirurgien utilise un premier bloc de coupe 40 qui, dans l'exemple de réalisation considéré ici, est fixé sur le tibia T par l'intermédiaire d'un guide de positionnement 42 : ce guide de positionnement 42 est conçu pour s'appuyer fixement sur le tibia T, notamment sur son épiphyse et/ou sa tubérosité antérieure, tout en permettant de régler au moins certaines caractéristiques du positionnement du bloc de coupe 40, supporté par le guide de positionnement 42, par rapport au tibia T. En particulier, avantageusement, le guide de positionnement 42 permet de régler la position du bloc de coupe 40 le long de la direction longitudinale du tibia T et/ou la position angulaire du bloc de coupe 40 autour d'un axe antéro-postérieur et/ou la position du bloc de coupe 40 le long d'un axe médio-latéral.

Bien entendu, à titre de variante non représentée, le guide de positionnement 42 décrit ci-dessus peut être remplacé par des moyens d'immobilisation non réglable du bloc de coupe 40 sur le tibia T, typiquement sous forme d'une ou de plusieurs broches d'ancrage osseux, liant directement et fixement le bloc de coupe et la matière osseuse du tibia.

Le bloc de coupe 40 délimite une fente 44 de passage d'un moyen de coupe osseuse, dans laquelle le chirurgien introduit et guide, par exemple, une lame de coupe 46, de manière à réséquer l'extrémité inférieure du tibia T selon un plan de coupe tibial correspondant.

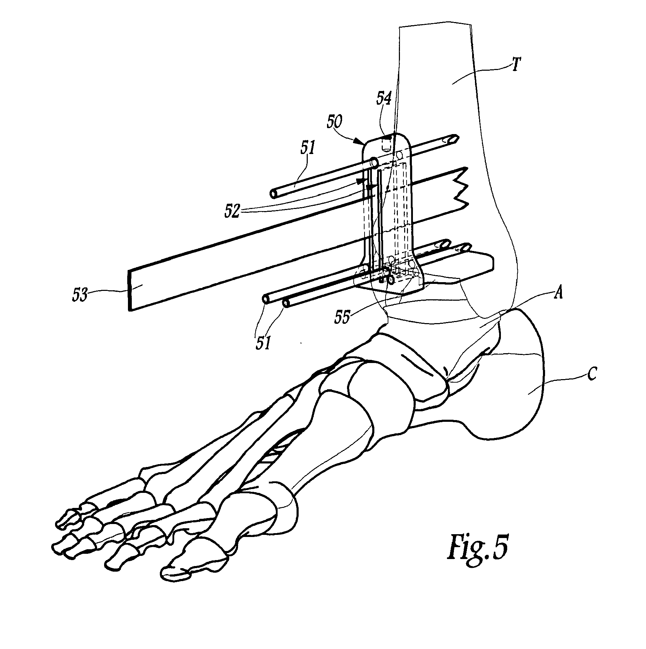

Avant de passer à la deuxième étape opératoire décrite et montrée à la figure 5, une étape intermédiaire optionnelle, non représentée, consiste à réséquer l'extrémité supérieure de l'astragale A de la cheville du patient, en formant alors un plan de coupe astragalien correspondant. En particulier, à titre d'exemple non limitatif, les plans de coupe tibial et astragalien sont sensiblement parallèles l'un à l'autre lorsque le pied du patient est positionné à 90° par rapport à sa jambe. En pratique, une telle résection de l'astragale A n'est souvent pas nécessaire dans le cadre d'une intervention chirurgicale de révision dans le sens où la préparation osseuse de l'astragale, qui avait été antérieurement réalisée aux fins de l'implantation d'une prothèse de cheville de première pose, a déjà donné lieu à la réalisation d'un tel plan de coupe astragalien et est donc généralement suffisante en vue de poser la prothèse de cheville 1. Ceci étant, en cas de besoin, le chirurgien a la possibilité d'utiliser une lame de coupe ou un moyen similaire pour réséquer l'extrémité supérieure de l'astragale A, avantageusement en introduisant et guidant cette lame de coupe dans la fente d'un bloc de coupe, qui est similaire au bloc de coupe 40 décrit plus haut et qui est avantageusement porté par le guide de positionnement 42.

Plus généralement, avant de passer à la deuxième étape opératoire décrite ci-après en regard de la figure 5, d'autres opérations secondaires de préparation osseuse de l'extrémité inférieure du tibia T et/ou de l'extrémité supérieure de l'astragale A peuvent être réalisées afin de disposer, sur ces extrémités, de plans de coupe appropriés à la pose de la prothèse de cheville 1, notamment en débarrassant les surfaces osseuses correspondant à ces plans de coupe d'éventuels ostéophytes ou de matières résiduelles.

Dans une deuxième étape opératoire, représentée à la figure 5, une fenêtre va être réalisée dans la face antérieure du tibia. Pour ce faire, le chirurgien utilise un second bloc de coupe 50 qu'il immobilise sur le tibia T, notamment au moyen de broches 51 introduites dans le tibia suivant une direction globalement antéro-postérieure. Ce bloc 50 délimite deux fentes 52, qui s'étendent parallèlement l'une à l'autre suivant une direction verticale et dans chacune desquelles le chirurgien introduit un moyen de coupe osseuse, en l'occurrence une lame de coupe 53 montrée à la figure 5, de manière à creuser dans la matière osseuse du tibia T deux rainures sensiblement verticales, qui ne débouchent que sur la face antérieure du tibia.

En pratique, avant d'immobiliser le bloc 50 sur le tibia T à l'aide des broches 51, le chirurgien positionne avec attention ce bloc 50 pour que, in fine, les deux rainures réalisées dans le tibia s'étendent parallèlement à la direction longitudinale du tibia T. Pour ce faire, à titre d'exemple, un élément de repérage 54 est prévu sur le bloc de coupe 50 pour que cet élément de repérage 54 soit aligné sur la crête tibiale dans le plan frontal à la cheville, tout en étant parallèle à cette crête dans un plan sagittal, lorsque le bloc de coupe est convenablement positionné par rapport au tibia. En complément, le bloc de coupe 51 est avantageusement pourvu, en saillie de sa face arrière, d'une palette 55 conçue pour être plaquée vers le haut contre le plan de coupe tibial.

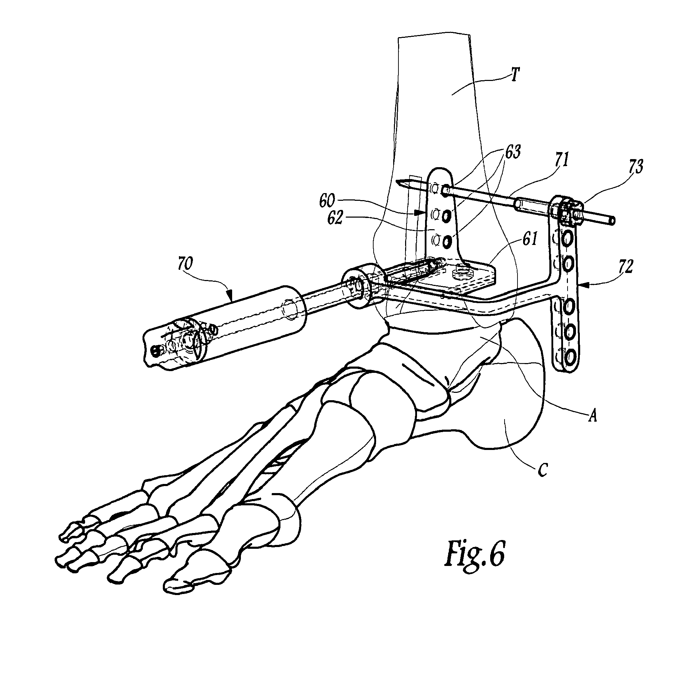

A l'issue de la deuxième étape opératoire, le bloc de coupe 50 est dégagé puis, à l'aide d'un ostéotome, en particulier d'un ostéotome de Poirier, le segment de matière osseuse, subsistant entre les deux rainures réalisées dans la face antérieure du tibia T, est détaché du reste du tibia, libérant ainsi une fenêtre à travers le tibia T : comme visible sur la figure 6, la fenêtre tibiale précitée débouche, à la fois, sur la face antérieure du tibia et sur le plan de coupe tibial. Si besoin, les parois délimitant la fenêtre tibiale précitée sont retravaillées, par exemple à l'aide de râpes prévues à cet effet.

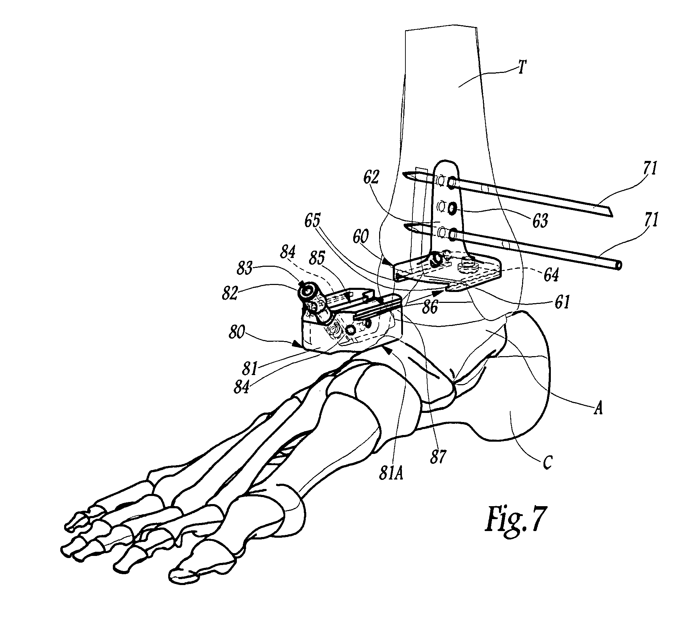

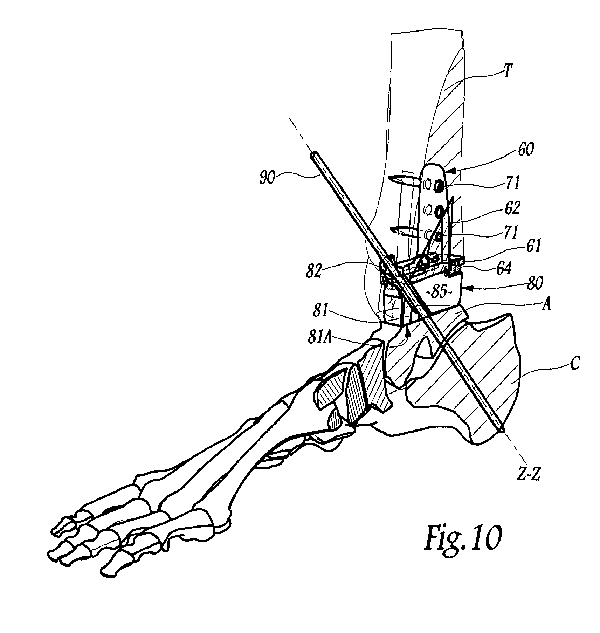

Dans une troisième étape opératoire, montrée à la figure 6, un implant tibial d'essai, autrement dit un fantôme 60 de l'implant tibial 10, est à mettre en place sur le tibia T. Avant d'expliquer les manipulations chirurgicales nécessaires, le fantôme tibial 60 est décrit ci-après. Ce fantôme tibial 60 comporte une plaque principale 61, qui présente un contour périphérique géométriquement analogue à celui de la plaque 11 de l'implant tibial 10 et dont le côté supérieur présente les mêmes aménagements que celui de la plaque 11, à savoir que la plaque 61 est pourvue d'une aile sagittale saillante 62 géométriquement analogue à l'aile 12 de l'implant tibial 10. En outre, l'aile 62 du fantôme tibial 60 délimite des trous traversants 63 de réception de broche de fixation au tibia. Sur son côté inférieur, la plaque 61 présente une surface plane qui, comme indiqué en pointillés à la figure 6 et comme partiellement visible sur les figures 7 et 10, est pourvue de:
- dans la région centrale de cette surface inférieure plane, un renflement saillant 64, qui, dans l'exemple de réalisation considéré ici, est venu de matière avec le reste de la plaque 61 et
- sur chacun des bords opposés, médial et latéral, de cette surface inférieure plane, une glissière 65, qui s'étend suivant une direction antéro-postérieure depuis l'extrémité avant de la plaque 61 vers l'extrémité arrière de celle-ci, et qui, dans l'exemple de réalisation considéré ici, présente un profil global en U, les évidements respectifs des profils en U des deux glissières 65 étant dirigés l'un vers l'autre suivant une direction médio-latérale.

Afin de mettre en place le fantôme tibial 60, le chirurgien le manipule à l'aide d'un préhenseur 70 dont l'extrémité distale est adaptée pour être fixée de manière amovible à une zone dédiée du fantôme tibial 60, cette zone étant située, pour le mode de réalisation considéré ici, à la base du côté antérieur de l'aile 62. On comprend qu'en déplaçant le préhenseur 70, le chirurgien est ainsi en mesure d'insérer le fantôme tibial 60 dans l'articulation de cheville du patient par voie antérieure, plus précisément en insérant l'aile 62 à l'intérieur de la fenêtre tibiale précitée, tout en plaquant la face supérieure de la plaque 61 contre le plan de coupe inférieur du tibia T.

En pratique, le chirurgien ajuste la position antéro-postérieure du fantôme tibial 60 par rapport au tibia T, en déplaçant de manière correspondante le préhenseur 70, tandis que, suivant la direction verticale, le chirurgien veille à ce que la plaque 61 soit maintenue en appui vers le haut contre le plan de coupe inférieur du tibia T, étant remarqué que le chirurgien peut être assisté pour cela par des écarteurs ou des matériels similaires.

Une fois que le chirurgien estime que le fantôme tibial 60 est correctement positionné sur le tibia T, il le fixe en position à l'aide de broches 71 introduites à travers l'extrémité inférieure du tibia T, en passant par l'un des trous traversants 63 de l'aile 62. Avantageusement, l'insertion de ces broches 71 est guidée par un guide de visée 72, qui est porté par le préhenseur 70 et qui porte un canon de perçage 73 pour guider chaque broche 71 selon les axes centraux respectifs des trous traversants 63.

Une fois que le fantôme tibial 60 est fixé sur le tibia T par les broches 71, le préhenseur 70 et le guide de visée 72 sont dégagés.

A titre optionnel, un contrôle par amplificateur de brillance peut alors être mis en oeuvre pour confirmer le bon positionnement du fantôme tibial 60 par rapport au tibia T.

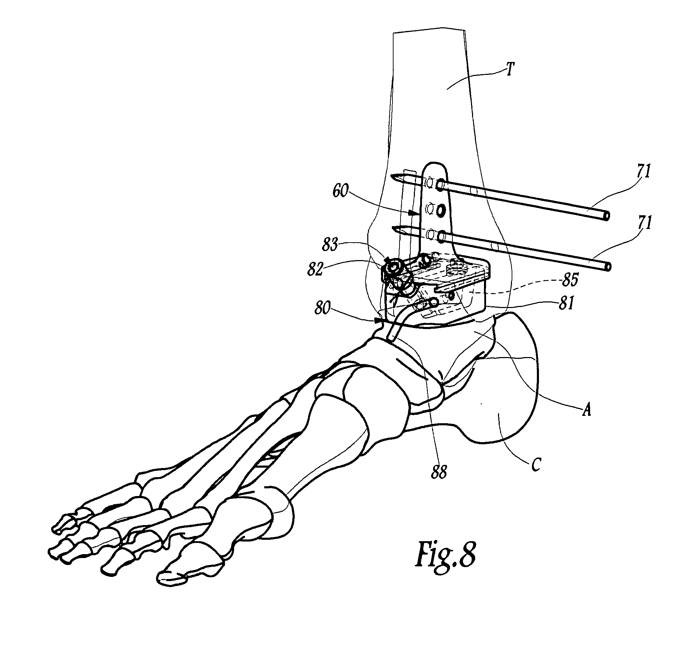

Dans une quatrième étape opératoire, qui est illustrée par les figures 7 à 10, l'astragale A et le calcanéum C vont être préparés en vue de positionner l'implantation de la quille astragalo-calcanéenne 22 de l'implant astragalien 20. Pour ce faire, le chirurgien utilise un guide de visée 80 qui, comme bien visible sur les figures 7 et 10, comporte un corps principal 81, ainsi qu'un canon de perçage 82 qui délimite un alésage 83 de passage de broche et qui est porté de manière amovible par le corps 81. Le corps 81 du guide de visée 80 est dimensionné pour être reçu de manière interposée, suivant la verticale, entre la plaque 61 du fantôme tibial 60 fixé au tibia T et le plan de coupe supérieur de l'astragale A, comme montré sur la figure 8. Plus précisément, le côté supérieur du corps principal 81 est pourvu, sur chacun de ces bords opposés, médial et latéral, d'un coulisseau 84 qui s'étend suivant une direction antéro-postérieure. Ces coulisseaux 84 sont adaptés pour être respectivement engagés de façon complémentaire dans les glissières 65 du fantôme tibial 60 de manière à assembler de manière réversible le fantôme tibial 60 et le guide de visée 80 selon une liaison coulissante antéro-postérieure.

Les glissières 65 et les coulisseaux 84 sont dimensionnés, à des jeux fonctionnels près, pour limiter, voire rendre sensiblement nuls les débattements relatifs entre le fantôme tibial 60 et le guide de visée 80 suivant, à la fois, la direction verticale et la direction médio-latérale. Cela revient à dire que la liaison coulissante antéro-postérieure entre le fantôme tibial 60 et le guide de visée 80 représente le seul degré de liberté de déplacement entre ces composants. En d'autres termes, le positionnement vertical et médio-latéral du fantôme tibial 60 sur le tibia T contraint de manière précise le positionnement vertical et médio-latéral du guide de visée 80.

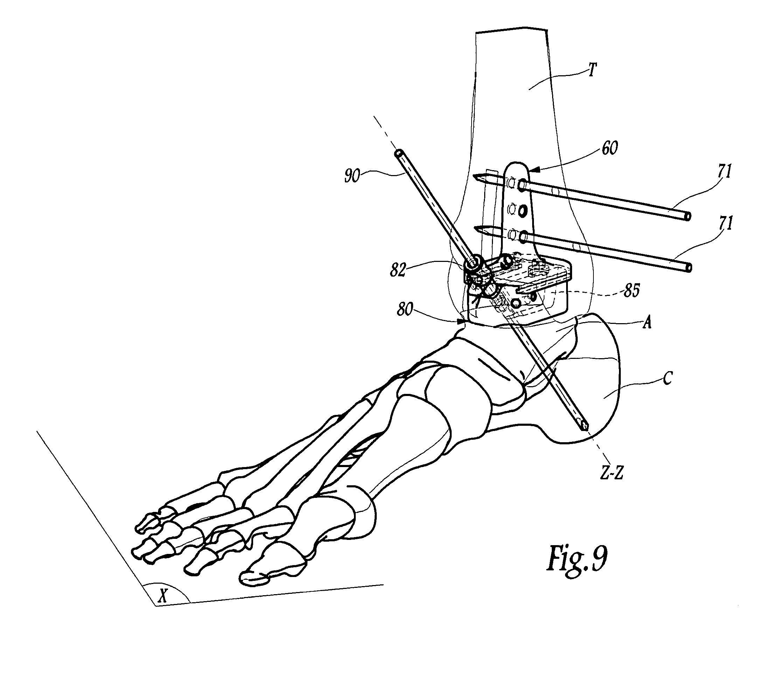

De plus, comme représenté partiellement en pointillés sur la figure 7 et comme bien visible à la figure 10, le corps principal 81 du guide de visée 80 délimite, dans sa région médiane, une fente 85, qui est ouverte sur l'extérieur en débouchant à la fois sur les faces opposées, supérieure et inférieure, du corps 81 et sur la face arrière de ce corps 81, tandis que la fente 85 est fermée au niveau de la face avant du corps 81 par une paroi avant de ce corps, aménagée pour y fixer de manière amovible le canon de perçage 82 de sorte que l'alésage 83 de ce dernier débouche vers le bas à l'intérieur de la fente 85. Ainsi, comme bien visible sur la figure 10, l'axe longitudinal central de l'alésage 83 traverse le corps 81 en s'étendant à l'intérieur de la fente 85, en allant de l'extrémité inférieure du canon de perçage 82 jusqu'à la face inférieure du corps principal 81. Pour des raisons qui apparaitront plus loin, on notera que le plan de la figure 10, qui correspond au plan noté X sur la figure 9, est un plan contenant l'axe central de l'alésage 83 et parallèle à la direction de coulissement de la liaison coulissante entre le fantôme tibial 60 et le guide de visée 80, ce plan formant un plan médian, notamment un plan de symétrie, pour la fente 85.

Dans le cadre de la méthode de pose de la prothèse de cheville 1 décrite jusqu'ici, le guide de visée 80 est manipulé par le chirurgien, le cas échéant, par l'intermédiaire d'un préhenseur non représenté, de façon à ce que le corps 81 de ce guide de visée est inséré sous le fantôme tibial 60, par coulissement du guide de visée 80 contre le fantôme tibial moyennant l'engagement des coulisseaux 84 dans les glissières 65, comme indiqué par la flèche 86 sur la figure 7. La position coulissée du guide de visée 80 par rapport au fantôme tibial 60, autrement dit le positionnement relatif antéro-postérieur entre le guide de visée et le fantôme tibial, est réglé par le chirurgien. Dans cette optique, plusieurs approches sont envisageables. En effet, ce réglage peut être laissé à l'entière discrétion du chirurgien. Ceci étant, un positionnement préférentiel ou extrême est avantageusement fourni au chirurgien, par mise en butée, suivant la direction antéro-postérieure, entre le renflement 64 de la plaque 61 du fantôme tibial 60 et le corps principal 81 du guide de visée 80, ce corps 81 délimitant d'ailleurs avantageusement un logement 87 de réception complémentaire du renflement 64 : à titre d'exemple, les figures 8 à 10 illustrent la mise en butée du renflement 64 dans ce logement 87.

Dans tous les cas, une fois que le positionnement relatif entre le guide de visée 80 et le fantôme tibial 60 est réglé conformément aux souhaits du chirurgien, et tout en maintenant le pied du patient à 90° de sa jambe, le chirurgien introduit une broche 90 dans le canon de perçage 82, cette broche étant entraînée vers l'arrière du pied, tout en étant guidé par l'alésage 83 de manière centrée sur l'axe de ce dernier, successivement à travers la fente 85, à travers tout l'astragale A et à travers au moins une partie, voire la totalité du calcanéum C, comme représenté sur les figures 9 et 10. Comme expliqué par la suite, cette broche astragalo-calcanéenne 90 détermine le positionnement d'implantation de l'implant astragalien 20, notamment en déterminant un axe Z-Z d'implantation de la quille 22 de cet implant astragalien 20.

A titre optionnel, juste avant de mettre en place la broche 90, le guide de visée 80 peut être utilisé par le chirurgien pour vérifier et, le cas échéant, ajuster l'alignement de l'articulation de la cheville alors que le pied du patient est maintenu à 90° de sa jambe. Pour ce faire, le guide de visée 80 est pourvu d'un relief ou une marque de repérage prédéterminé de la position du guide de visée 80 par rapport à au moins une singularité anatomique de l'astragale A, telle que l'articulation astragalo-naviculaire : d'ailleurs, sur la figure 8, le corps principal 81 porte, à titre d'aménagement optionnel avantageux, un tel relief de repérage 88 sur lequel le chirurgien peut aligner l'articulation astragalo-naviculaire du pied.

A l'issue de cette quatrième étape opératoire, alors que la broche 90 est laissée en place à travers l'astrale A et le calcanéum C, et alors que le fantôme tibial 60 est laissé en place sur le tibial T, le chirurgien dégage totalement le guide de visée 80. Plus précisément, le chirurgien désolidarise le canon de perçage 82 vis-à-vis du corps principal 81 puis fait coulisser ce canon de perçage le long de la broche 90 en direction de l'extrémité avant de cette dernière, jusqu'à l'ôter de la broche. Puis, le chirurgien fait coulisser vers l'avant le corps principal 81 vis-à-vis de fantôme tibial 60 : on comprend alors l'intérêt de la présence de la fente 85 puisque celle-ci va permettre le dégagement du corps principal 81 sans que ce dernier interfère avec la broche 90 laissée en place, cette fente 85 étant dimensionnée en conséquence, notamment en ce qui concerne son inclinaison par rapport aux faces supérieure et inférieure du corps principal 81, ainsi qu'en ce qui concerne sa largeur médio-latérale.

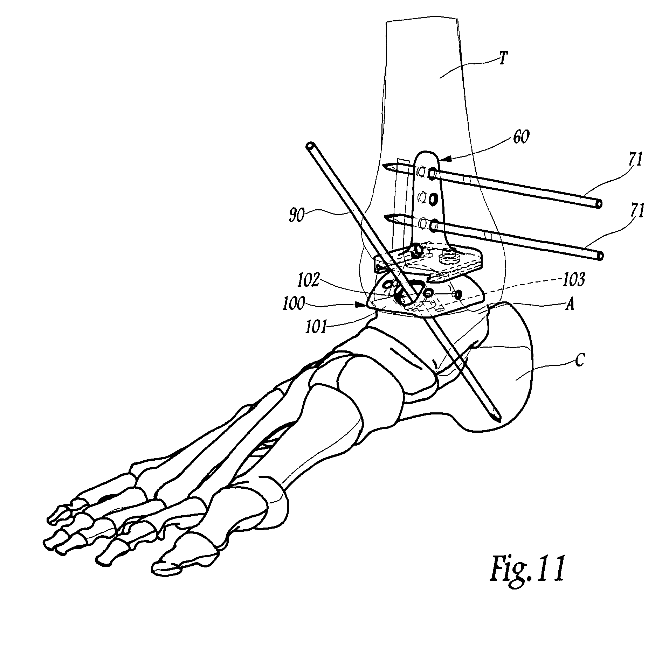

Dans une cinquième étape opératoire illustrée par les figures 11 à 13, l'astragale A et le calcanéum C vont être percés pour réaliser dans ces os un logement de réception complémentaire de la quille 22 de l'implant astragalien 20. Pour ce faire, comme représenté sur la figure 11, un composant astragalien d'essai, autrement dit un fantôme astragalien 100 est mis en place de manière interposée entre le fantôme tibial 60 et l'astragale A. Ce fantôme astragalien 100 comporte un bloc principal 101 délimitant une surface inférieure plane qui présente un contour périphérique géométriquement analogue à celui de la surface inférieure du bloc principal 21 de l'implant astragalien 20 : on comprend que ce bloc 101 est mis en place sur l'astragale A, en le plaquant contre le plan de coupe supérieur de cet astragale, autrement dit dans une configuration similaire à celle que va occuper l'implant astragalien 20 à l'issue de l'intervention.

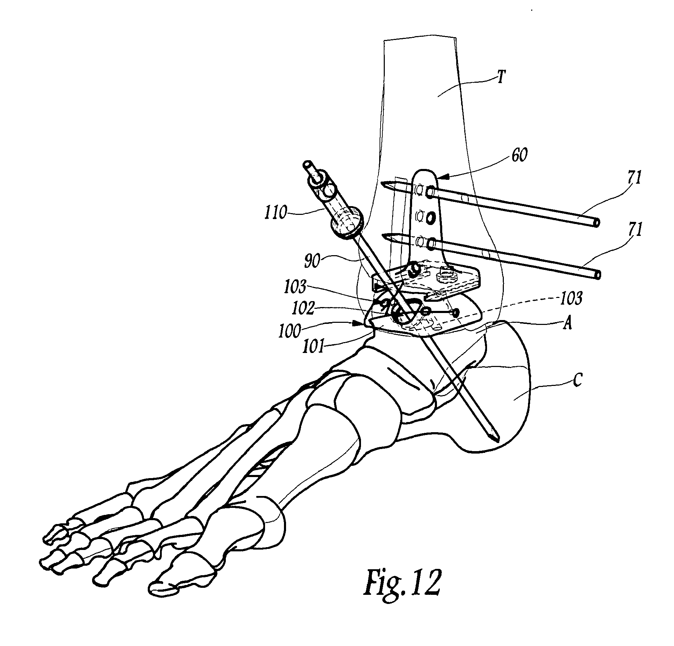

On notera que le fantôme astragalien 100 est ainsi mis en place alors que la broche 90 est laissée en place à travers l'astragale A et le calcanéum C. Par conséquent, le bloc principal 101 est pourvu d'un trou traversant 102, qui relie les côtés supérieur et inférieur du bloc 101 et à l'intérieur duquel est engagée axialement la broche 90 : autrement dit, le fantôme astragalien 100 est glissé sur la broche 90 jusqu'à plaquer la face inférieure de son bloc principal 101 contre le plan de coupe de l'astragale A. On comprend qu'une certaine liberté d'inclinaison du bloc principal 101 par rapport à la broche 90 doit être laissée, ce qui explique pourquoi le diamètre du trou traversant 102 est significativement plus grand que le diamètre externe de la broche 90. Cette disposition peut obliger le chirurgien à utiliser ensuite un centreur 110 qui, comme représenté sur la figure 12, est glissé sur la broche 90, en étant ajusté sur le diamètre extérieur de celle-ci, jusqu'à être agencé, en ajustement, de manière radialement interposée entre la broche 90 et le bloc principal 101, au niveau du trou traversant 102 de ce dernier. Une fois que le centreur 110 occupe le trou traversant 102, le positionnement du fantôme astragalien 100 est contraint de manière précise vis-à-vis de la broche 90, étant rappelé que le bloc 101 de ce fantôme est maintenu en appui vers le bas contre le plan de coupe supérieure de l'astragale. La position du fantôme astragalien 100 peut alors être figée vis-à-vis de l'astragale, en fixant le bloc principal 101 à l'aide de deux broches rapportées, non représentées, introduites dans des trous traversants dédiés 103 du bloc principal 101.

A titre de variante non représentée, le trou traversant 102 débouche sur l'avant du bloc 101, ce qui revient à dire que ce trou traversant forme une fente ouverte à l'une de ses extrémités : cela facilite la mise en place de la broche 90 dans cette fente, dans le sens où, en tout niveau intermédiaire de la broche, cette dernière peut être engagée latéralement dans la fente précitée. A moins de limiter la largeur de la fente précitée au diamètre de la broche 90, ce qui peut s'avérer contraignant en termes de manipulations peropératoires, l'utilisation subséquente du centreur 110 reste pertinente.

A titre optionnel, avant de poursuivre le déroulement de la cinquième étape opératoire, un contrôle par amplificateur de brillance peut à ce stade confirmer le bon alignement des fantômes tibial 60 et astragalien 100.

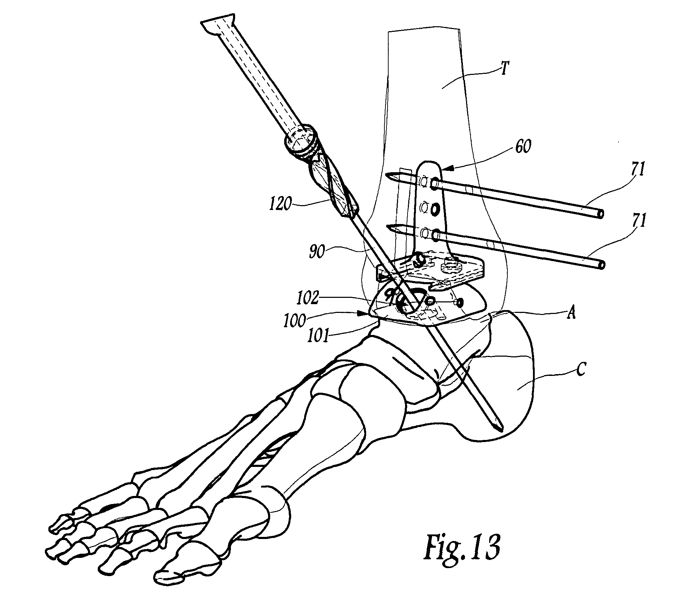

Après avoir retiré le centreur 110 en l'entraînant le long de la broche 90 suivant une manipulation inverse à sa mise en place, l'astragale A et le calcanéum C sont percés pour réaliser dans ces os un logement de réception complémentaire de la quille 22 de l'implant astragalien 20. Ce perçage est avantageusement réalisé à l'aide de la broche 90 laissée en place à travers l'astragale A et le calcanéum C, en utilisant cette broche 90 comme support de guidage le long duquel est coulissé au moins un outil de perçage osseux 120, tel qu'une fraise canulée ou un alésoir, comme représenté sur la figure 13. D'ailleurs, plusieurs de ces outils de perçage peuvent être utilisés de manière successive pour élargir et/ou retravailler la forme des perçages osseux successifs. Dans tous les cas, le bloc principal 101 forme une butée, selon la direction longitudinale de la broche 90, pour ce ou ces outils de perçage 120 : de cette façon, l'axe longitudinal central de la broche 90 détermine l'axe central Z-Z autour duquel est réalisé le logement de réception de la quille d'ancrage 22, tandis que la mise en butée du ou des outils de perçage 120 contre le fantôme astragalien 100 détermine la profondeur de ce logement.

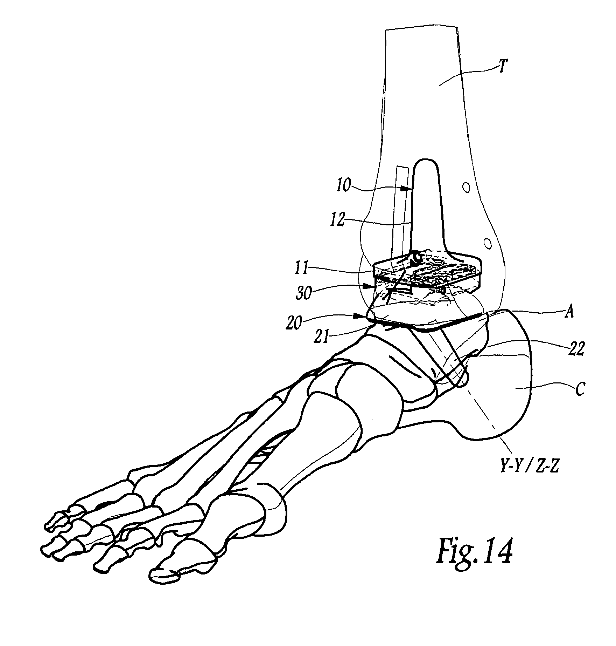

A ce stade de l'intervention chirurgicale, le tibia T, l'astragale A et le calcanéum C sont prêts à recevoir les implants tibial 10 et astragalien 20 de la prothèse 1, comme décrit ci-après en regard de la figure 14. A titre optionnel, avant de mettre en place ces implants 10 et 20, des essais peuvent être réalisés par le chirurgien avec le fantôme tibial 60, le fantôme astragalien 100, et un ou plusieurs fantômes, non représentés, du patin 30 pour ajuster l'écartement vertical entre l'extrémité inférieure du tibia T et l'extrémité supérieure de l'astragale A et ainsi rétablir un écartement anatomique entre elles, ainsi qu'avec des tiges d'essais, non représentées, passées à travers le bloc principal 101, via le trou traversant 102, pour vérifier la bonne profondeur du logement réalisé à travers l'astragale et le calcanéum. Une fois ces essais terminés, la broche 90, ainsi que les broches 71 sont retirées et les fantômes 60 et 100 sont dégagés.

L'intervention chirurgicale prend fin avec la mise en place des implants tibial 10 et astragalien 20, comme représenté sur la figure 14. En particulier, l'implant astragalien 20 est monté sur un préhenseur spécifique, non représenté, afin d'être mis en place sur l'astragale A, en engageant sa quille 22 dans le logement réalisé à travers l'astragale A et le calcanéum C et en alignant son axe Y-Y sur l'axe Z-Z pour l'implanter selon cet axe. Puis, l'implant tibial 10, sur lequel a été préalablement fixé le patin prothétique 30, est, notamment à l'aide d'un préhenseur spécifique non représenté, mis en place sur le tibia T, en occupant, in fine, la même configuration de fixation au tibia qu'occupait le fantôme tibial 60.

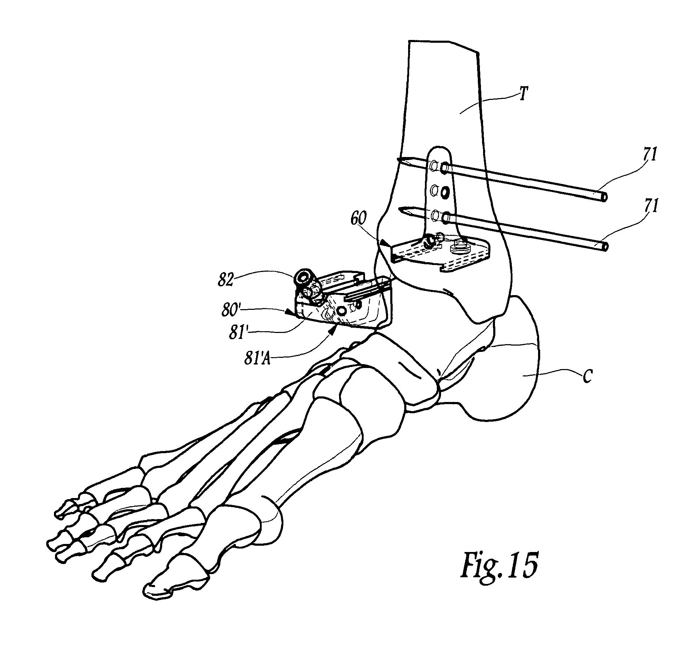

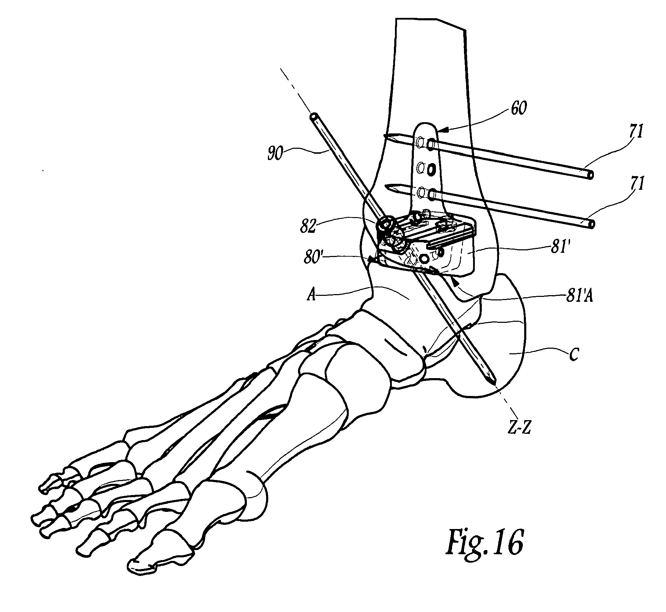

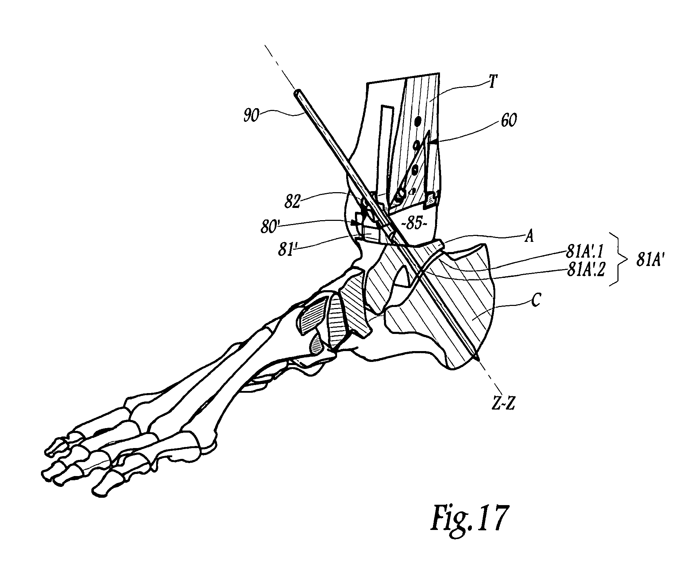

Sur les figures 15 à 17 est représentée une variante de l'instrumentation chirurgicale décrite jusqu'ici, qui ne se distingue de cette dernière que par son guide de visée, référencé 80'. Plus précisément, comme bien visible par comparaison des figures 15, 16 et 17 avec les figures 7, 9 et 10, le guide de visée 80' est identique au guide de visée 80, excepté pour ce qui concerne le côté inférieur de son corps principal 81'. Aussi, sur les figures 15 à 17, les composants identiques à ceux montrés sur les figures 7, 9 et 10 portent les mêmes références numériques et ne seront pas décrits ici plus avant, le lecteur pouvant se reporter à la description détaillée fournie plus haut.

Ainsi, à la différence du corps 81 du guide de visée 80, qui, sur son côté inférieur, délimite une face inférieure 81A sensiblement plane et orientée suivant une direction antéropostérieure, le corps 81' du guide de visée 80' présente, sur son côté inférieur, une face inférieure 81A' de forme globalement biseautée : dans l'exemple de réalisation considéré sur les figures 15 à 17, cette face inférieure 81A' inclut une partie postérieure 81A'.1, qui est sensiblement plane et orientée suivant une direction antéropostérieure, ainsi qu'une partie antérieure 81A'.2, qui est sensiblement plane et qui relie, de manière inclinée par rapport à la direction antéropostérieure, la partie postérieure 81A'.1 et la face antérieure du corps 81'. Cette différence de conformation des faces inférieures 81A et 81A' des corps 81 et 81' est liée à des considérations relatives à l'état osseux dans lequel se trouve l'astragale A. En effet, comme expliqué en détail plus haut, l'utilisation du guide de visée 80 présuppose la présence d'un plan de coupe supérieur de l'astragale A, ce plan de coupe supérieur étant soit pré-existant à la mise en place de la prothèse de cheville 1, soit spécifiquement réalisé par le chirurgien au début de l'intervention chirurgicale visant à implanter cette prothèse de cheville 1 : dans ce cas, on comprend que, au cours de l'intervention chirurgicale, le chirurgien fait facilement coopérer, par un contact plan-plan, le plan de coupe supérieur précité de l'astragale A et la face inférieure 81A du corps 81 du guide de visée 80, notamment à des fins d'ajustement de leur position relative, en particulier lors de la mise en place de la broche astragalo-calcanéenne 90. Ceci étant, dans le cas où l'astragale A présente une altération telle que la réalisation d'un tel plan de coupe supérieur est rendue impossible, notamment du fait d'une nécrose ou d'un endommagement de l'astragale, la préparation osseuse de l'astragale est alors réalisée par le chirurgien à un niveau anatomique situé plus près du calcanéum C, tout du moins pour la partie postérieure de l'astragale, tandis que la partie antérieure de l'astragale est généralement restaurée par un greffon osseux : on comprend ainsi que, d'une part, la partie postérieure 81A'.1 de la face inférieure 81A' du corps 81' du guide de visée 80' peut être mise en coopération, suivant un contact plan-plan, avec la partie arrière résiduelle de l'astragale A, voire directement avec le calcanéum C dans le cas où la partie arrière de l'astragale A est totalement retirée par le chirurgien, et, d'autre part, la partie antérieure 81A'.2 de cette face inférieure 81A' peut alors être utilisée par le chirurgien pour coopérer avec le greffon osseux précité, le cas échéant en reposant contre ce greffon de manière à le stabiliser.

Bien entendu, pour la forme de réalisation de l'instrumentation illustrée par les figures 15 à 17, on comprend que le fantôme astragalien correspondant, non représenté sur ces figures, a son bloc principal avec une face inférieure géométriquement similaire à la face inférieure 81A' du corps 81'.

Plus généralement, à titre de variante non représentée, on comprend que la géométrie des faces inférieures du corps du guide de visée et du corps du fantôme astragalien peut être adaptée à une préparation osseuse particulière de l'astragale A, tenant compte notamment de l'état de la matière osseuse de cette dernière.

Divers aménagements et variantes à l'instrumentation chirurgicale décrite jusqu'ici, ainsi qu'à la méthode d'utilisation de cette instrumentation, sont par ailleurs envisageables. A titre d'exemples :
- d'autres formes de réalisation que la liaison coulissante sont possibles pour la liaison mécanique entre le fantôme tibial 60 et le guide de visée 80 ou 80' du moment que cette liaison mécanique prédétermine le positionnement relatif du guide de visée et du fantôme tibial alors que ce dernier est fixé au tibia ;
- après que la broche 90 a été mise en place, autrement dit après la quatrième étape opératoire décrite plus haut, le chirurgien peut, pour diverses raisons, souhaiter poursuivre l'intervention en décalant l'axe selon lequel la quille d'ancrage 22 de l'implant astragalien 20 sera implantée à l'issue de l'intervention ; pour ce faire, l'instrumentation comporte avantageusement une pièce de décalage ad hoc, sous forme d'un bloc traversé par au moins deux trous parallèles l'un à l'autre ; cette pièce de décalage est rapportée sur la broche 90 déjà implantée, en engageant cette dernière dans l'un des deux trous précités, tandis que l'autre trou est alors utilisé par le chirurgien pour mettre en place une seconde broche astragalo-calcanéenne, similaire à la broche 90 ; on comprend que cette autre broche astragalo-calcanéenne s'étend alors parallèlement à la broche 90, tout en étant décalée par rapport à cette dernière de l'entraxe prédéterminé entre les deux trous de la pièce de décalage ; dans le prolongement des considérations qui précèdent, une forme de réalisation avantageuse de cette pièce de décalage consiste en un barillet incluant plusieurs trous traversants, structurellement et fonctionnellement similaires aux deux trous précités et répartis de manière prédéterminée les uns par rapport aux autres ; et/ou
- plutôt que d'utiliser la broche 90, d'autres éléments d'instrumentation fonctionnellement similaires peuvent être employés.

## Revendications

1. Ensemble d'instrumentation chirurgicale pour poser une prothèse de cheville (1), ladite prothèse de cheville incluant un implant tibial (10) et un implant astragalien (20) pourvu d'une quille d'ancrage astragalo-calcanéen (22),
cet ensemble d'instrumentation comportant :
- un fantôme tibial (60) de l'implant tibial, adapté pour être fixé au tibia (T) d'un patient, et
- un guide de visée (80 ; 80'), adapté pour mettre en place un élément d'instrumentation (90) à travers l'astragale (A) et le calcanéum (C) du patient, selon un axe (Z-Z) d'implantation de la quille d'ancrage astragalo-calcanéen, **caracterisé en ce que** le fantôme tibial (60) et le guide de visée (80 ; 80') sont conçus pour coopérer mécaniquement l'un avec l'autre de manière à, alors que le fantôme tibial est fixé au tibia (T), assembler le guide de visée au fantôme tibial, en fixant, à des jeux fonctionnels près, leur positionnement relatif suivant des directions verticale et médio-latérale, puis utiliser ce guide de visée pour mettre en place l'élément d'instrumentation (90).

2. Ensemble d'instrumentation suivant la revendication 1, **caractérisé en ce que** le guide de visée (80) définit un passage traversant (85) de réception de l'élément d'instrumentation (90), ce passage étant conçu pour dégager le guide de visée vis-à-vis du fantôme tibial (60) fixé au tibia (T) tout en laissant l'élément d'instrumentation en place à travers l'astragale (A) et le calcanéum (C).

3. Ensemble d'instrumentation suivant la revendication 2, **caractérisé en ce que** le guide de visée (80 ; 80') comporte à la fois :
- un corps principal (81 ; 81'), qui délimite le passage (85) et qui, en service, coopère mécaniquement avec le fantôme tibial (60) aux fins du positionnement fixe, à la fois vertical et médio-latéral, entre le guide de visée et le fantôme tibial, et
- un canon de perçage (82), qui est fixé de manière amovible sur le corps (81), en débouchant dans le passage (85), et qui est adapté pour guider l'élément d'instrumentation (90) selon l'axe (Z-Z) d'implantation de la quille d'ancrage astragalo-calcanéen (22).

4. Ensemble d'instrumentation suivant la revendication 3, **caractérisé en ce que** le passage inclut, voire consiste en une fente (85) qui débouche à la fois sur les faces du corps principal (81, 81'), opposées selon la direction longitudinale de l'élément d'instrumentation (90), et sur au moins une autre face du corps principal aux fins du dégagement du guide de visée (80 ; 80') vis-à-vis du fantôme tibial (60) tout en laissant l'élément d'instrumentation en place.

5. Ensemble d'instrumentation suivant l'une quelconque des revendications précédentes, **caractérisé en ce que** le fantôme tibial (60) et le guide de visée (80 ; 80') sont pourvus de moyens mécaniques (65, 84) de coopération l'un avec l'autre, adaptés pour assembler de manière réversible le fantôme tibial et le guide de visée l'un à l'autre selon une liaison essentiellement coulissante qui s'étend suivant une direction antéro-postérieure.

6. Ensemble d'instrumentation suivant la revendication 5, **caractérisé en ce que** le fantôme tibial (60) et le guide de visée (80 ; 80') sont pourvus de moyens (64, 87) de butée d'arrêt relatif entre eux selon la direction de coulissement de la liaison coulissante.

7. Ensemble d'instrumentation suivant l'une quelconque des revendications précédentes, **caractérisé en ce que** le guide de visée (80 ; 80') est pourvu d'un relief (88) ou d'une marque de repérage de sa position par rapport à une singularité anatomique de l'astragale (A), en particulier par rapport à l'articulation astragalo-naviculaire.

8. Ensemble d'instrumentation suivant l'une quelconque des revendications précédentes, **caractérisé en ce que** l'ensemble d'instrumentation comporte en outre un fantôme astragalien (100) de l'implant astragalien, qui est adapté pour, alors que l'élément d'instrumentation (90) est en place à travers l'astragale (A) et le calcanéum (C), être engagé sur cet élément d'instrumentation jusqu'à une position d'appui contre l'astragale, position dans laquelle le fantôme astragalien est adapté pour former une butée, selon l'axe (Z-Z) d'implantation de la quille d'ancrage astragalo-calcanéen, pour un outil (120) de perçage dans l'astragale et le calcanéum d'un logement de réception de la quille d'ancrage astragalo-calcanéen (22), centré sur l'axe d'implantation (Z-Z).

9. Ensemble d'instrumentation selon la revendication 8, **caractérisé en ce que** l'ensemble d'instrumentation comporte en outre un centreur (110) qui est adapté pour être rapporté autour de l'élément d'instrumentation (90), en étant radialement interposé entre cet élément d'instrumentation, en place à travers l'astragale (A) et le calcanéum (C), et le fantôme astragalien (100) dans sa position d'appui contre l'astragale.

10. Ensemble d'instrumentation suivant l'une quelconque des revendications précédentes, **caractérisé en ce que** l'ensemble d'instrumentation comporte en outre :
- des moyens (40, 42, 44, 46) de résection de l'extrémité inférieure du tibia (T), avantageusement adaptés pour régler la position, par rapport au tibia, d'un plan de coupe tibial correspondant, et
- des moyens (70, 71, 72, 73) de mise en place et de fixation du fantôme tibial (60) sur le tibia (T), adaptés pour plaquer au moins une partie dédié (61) du fantôme tibial contre le plan de coupe tibial.

## Patentansprüche

1. Chirurgischer Instrumentierungssatz zum Positionieren einer Knöchelprothese (1), wobei die Knöchelprothese ein Tibiaimplantat (10) und ein Talusimplantat (20) aufweist, das mit einem Talus-Calcaneus-Verankerungskiel (22) versehen ist,
wobei der Instrumentierungssatz aufweist:
- ein Tibiaphantom (60) des Tibiaimplantats, das angepasst ist, an der Tibia (T) eines Patienten befestigt zu sein, und
- eine Zielführung (80, 80'), die angepasst ist, ein Instrumentierungselement (90) durch den Talus (A) und den Calcaneus (C) des Patienten hindurch entlang einer Implantationsachse (Z-Z) des Talus-Calcaneus-Verankerungskiels in Postition zu bringen, **dadurch gekennzeichnet, dass**
das Tibiaphantom (60) und die Zielführung (80, 80') eingerichtet sind, mechanisch zusammenzuwirken, um, wenn das Tibiaphantom an der Tibia (T) befestigt ist, die Zielführung an dem Tibiaphantom zu montieren, wobei ihre relative Positionierung entlang einer vertikalen und einer mediolateralen Richtung mit engem funktionellem Spiel fixiert wird, und anschließend die Zielführung zu verwenden, um das Instrumentierungselement (90) in Position zu bringen.

2. Instrumentierungssatz gemäß Anspruch 1, **dadurch gekennzeichnet, dass** die Zielführung (80) einen Durchgang (85) zum Aufnehmen des Instrumentierungselements (90) definiert, wobei der Durchgang eingerichtet ist, die Zielführung bezüglich des an der Tibia (T) befestigten Tibiaphantoms (60) zu lösen, wobei das Instrumentierungselement durch den Talus (A) und den Calcaneus (C) hindurch in Position gelassen wird.

3. Instrumentierungssatz gemäß Anspruch 2, **dadurch gekennzeichnet, dass** die Zielführung (80, 80') gleichzeitig aufweist:
- einen Hauptkörper (81, 81'), der den Durchgang (85) begrenzt, und der im Betrieb mechanisch mit dem Tibiaphantom (60) zusammenwirkt, zum Zweck der gleichzeitig vertikalen und mediolateralen festen Positionierung zwischen der Zielführung und dem Tibiaphantom, und
- eine Bohrbuchse (82), die auf lösbare Weise an dem Körper (81) befestigt ist, wobei sie in den Durchgang (85) mündet, und die angepasst ist, das Instrumentierungselement (90) entlang der Implantationsachse (Z-Z) des Talus-Calcaneus-Verankerungskiels (22) zu führen.

4. Instrumentierungssatz gemäß Anspruch 3, **dadurch gekennzeichnet, dass** der Durchgang einen Schlitz (85) aufweist bzw. aus einem solchen besteht, der gleichzeitig an den Seiten des Hauptkörpers (81, 81'), die einander entlang der Längsrichtung des Instrumentierungselements (90) entgegengesetzt sind, und an mindestens einer weiteren Seite des Hauptkörpers einmündet, zum Zweck des Lösens der Zielführung (80, 80') bezüglich des Tibiaphantoms (60), wobei das Instrumentierungselement in Position gelassen wird.

5. Instrumentierungssatz gemäß irgendeinem der vorhergehenden Ansprüche, **dadurch gekennzeichnet, dass** das Tibiaphantom (60) und die Zielführung (80, 80') mit
mechanischen Mitteln (65, 84) zum miteinander Zusammenwirken versehen sind, die angepasst sind, das Tibiaphantom und die Zielführung gemäß einer im Wesentlichen gleitenden Verbindung, die sich entlang einer anterior-posterioren Richtung erstreckt, auf reversible Weise zu montieren.

6. Instrumentierungssatz gemäß Anspruch 5, **dadurch gekennzeichnet, dass** das Tibiaphantom (60) und die Zielführung (80, 80') mit Anschlagsmitteln (64, 87) für eine relative Arretierung untereinander entlang der Gleitrichtung der Gleitverbindung versehen sind.

7. Instrumentierungssatz gemäß irgendeinem der vorhergehenden Ansprüche, **dadurch gekennzeichnet, dass** die Zielführung (80, 80`) mit einem Relief (88) oder mit einer Markierung zum Kennzeichnen ihrer Position bezüglich einer anatomischen Eigenart des Talus (A), insbesondere bezüglich des Talonavikulargelenks versehen ist.

8. Instrumentierungssatz gemäß irgendeinem der vorhergehenden Ansprüche, **dadurch gekennzeichnet, dass** der Instrumentierungssatz ferner ein Talusphantom (100) des Talusimplantats aufweist, das angepasst ist, wenn das Instrumentierungselement (90) durch den Talus (A) und den Calcaneus (C) hindurch in Position ist, bis zu einer Position des Anstoßens an den Talus an dem Instrumentierungselement anzugreifen, wobei dies eine Position ist, in der das Talusphanto angepasst ist, entlang der Implantationsachse (Z-Z) des Talus-Calcaneus-Verankerungskiels einen Anschlag auszubilden, für ein Werkzeug (120) zum Bohren einer Aufnahme zum Aufnehmen des Talus-Calcaneus-Verankerungskiels (22) in den Talus und den Calcaneus, die an der Implantationsachse (Z-Z) zentriert ist.

9. Instrumentierungssatz gemäß Anspruch 8, **dadurch gekennzeichnet, dass** der Instrumentierungssatz ferner eine Zentrierungsvorrichtung (110) aufweist, die angepasst ist, uran das Instrumentierungselement (90) herum angefügt zu werden, wobei sie radial zwischen dem Instrumentierungselement, das durch den Talus (A) und den Calcaneus (C) hindurch in Position ist, und dem Talusphantom (100) in seiner an den Talus anstoßenden Position positioniert wird.

10. Instrumentierungssatz gemäß irgendeinem der vorhergehenden Ansprüche, **dadurch gekennzeichnet, dass** der Instrumentierungssatz ferner aufweist:
- Mittel (40, 42, 44, 46) für die Resektion des unteren Endes der Tibia (T), die vorteilhafterweise angepasst sind, die Position einer korrespondierenden Tibiaschnittebene bezüglich der Tibia einzustellen, und
- Mittel (70, 71, 72, 73) zum Platzieren und Fixieren des Tibiaphantoms (60) an der Tibia (T), die angepasst sind, indestens einen spezifischen Teil (61) des Tibiaphantoms gegen die Tibiaschnittebene zu pressen.

## Claims

1. Surgical instrumentation kit for positioning an ankle prosthesis (1), wherein said ankle prosthesis includes a tibial implant (10) and an astragalar implant (20) equipped with an astragalo-calcaneal anchoring rod (22),
said instrumentation kit comprising:
- a tibial phantom (60) of the tibial implant, suitable for being attached to the tibia (T) of a patient, and
- an aiming guide (80; 80'), suitable for placing an instrumentation element (90) through the astragalus (A) and the calcaneus (C) of the patient, according to an axis (Z-Z) of implantation of the astragalo-calc eal choring rod,
**characterized in that** the tibial phantom (60) and the aiming guide (80; 80') are designed so as to cooperate mechanically one with the other so as to assemble, while the tibial phantom is fixed to the tibia (T), the aiming guide to the tibial phantom, by fixing, with close functional clearances, their relative positioning according to the vertical and mediolateral directions, then using this aiming guide to placing the instrumentation element (90).

2. Instrumentation kit according to claim 1, **characterized in that** the aiming guide (80) defines a through-passage (85) for receiving the instrumentation element (90), this passage being designed so as to release the aiming guide from the tibial phantom (60) fixed to the tibia (T) while leaving the instrumentation element in place through the astragalus (A) and the calcaneus (C).

3. Instrumentation kit according to claim 2, **characterized in that** the aiming guide (80; 80') comprises:
- a main body (81; 81'), which delimits the passage (85) and which, during operation, cooperates mechanically with the tibial phantom (60) for the purpose of stationary positioning, both vertical and mediolateral, between the aiming guide and the tibial phantom, and
- a drill bush (82), which is removably attached to the body (81), leading into the passage (85), and which is suitable for guiding the instrumentation element (90) according to the axis (Z-Z) of implantation of the astragalo-calcaneal choring rod (22).

4. Instrumentation kit according to claim 3, **characterized in that** the passage includes, or even consists of, a slot (85) that leads both to the faces of the main body (81, 81'), which are mutually opposite according to the longitudinal direction of the instrumentation element (90), to at least one other face of the main body for the purpose of releasing the aiming guide (80; 80') with respect to the tibial phantom (60) while leaving the instrumentation element in place.

5. Instrumentation kit according to any one of the previous claims, **characterized in that** the tibial phantom (60) and the aiming guide (80; 80') are equipped with mechanical means (65, 84) for cooperation with one another, suitable for reversibly assembling the tibial phantom and the aiming guide to one other according to a substantially sliding connection that extends in an anteroposterior direction.

6. Instrumentation kit accordin to claim 5, **characterized in that** the tibial phantom (60) and the aiming guide (80; 80') are equipped with means (64, 87) serving as a mutual relative end stop according to the sliding direction of the sliding connection.

7. Instrumentation kit according to any one of the previous claims, **characterized in that** the aiming guide (80; 80') is equipped with a relief (88) or a mark for identifying its position with respect to an anatomical feature of the astragalus (A), in particular with respect to the as agalo-navicular joint.

8. Instrumentation kit according to any one of the previous claims, **characterized in that** the instrumentation kit also comprises an astragalar phantom (100) of the astragalar implant, which is suitable for, when the instrumentation kit (90) is in place through the astragalus (A) and the calcaneus (C), being engage on this instrumentation element until a bearing position against the astragalus, a position in which the astragalar phantom is suitable for forming a stop, according to the implantation axis (Z-Z) of the astragalo-calcaneal anchoring rod, for a tool (120) for drilling, in the astragalus and the calcaneus, a recess for receiving the astragalo-calcaneal anchoring rod (22), centred on the implantation axis (Z-Z).

9. Instrumentation kit according to claim 8, **characterized in that** the instrumentation kit also comprises a centring device (110) that is suitable for being attached around the instrumentation element (90), by being radially inserted between this instrumentation element, in place through the astragalus (A) and the calcaneus (C), and the astragalar phantom (100) in its bearing position against the astragalus.

10. Instrumentation kit according to any one of the previous claims, **characterized in that** the instrumentation kit also comprises:
- means (40, 42, 44, 46) for resecting the lower end of the tibia (T), advantageously suitable for adjusting the position, with respect to the tibia, of a corresponding tibial cutting plane, and
- means (70, 71, 72, 73) for placing and attaching the tibial phantom (60) on the tibia (T), suitable for pressing at least one dedicated portion (61) of the tibial phantom against the tibial cutting plane.
